# EUROPEAN PATENT APPLICATION

(11) **EP 3 165 265 A1**
(43) Date of publication of application: **10.05.2017**
(21) Application number: 16196761.7
(22) Date of filing: 01.11.2016
(51) Int. Cl.: A63F 13/212, A63F 13/65, A63F 13/67, A61B 90/00

(54) **SYSTEM AND METHOD FOR ENABLING A USER TO OVERCOME WEAK BEHAVIORAL TRAITS**

(30) Priority: 04.11.2015 US 201514931868; 06.11.2015 US 201514934150; 21.12.2015 US 201514975879; 21.12.2015 US 201514975881; 21.12.2015 US 201514975885; 21.12.2015 US 201514975888; 31.12.2015 US 201514985472; 31.12.2015 US 201514985475; 31.12.2015 US 201514985478; 21.01.2016 US 201615002416; 21.01.2016 US 201615002417; 21.01.2016 US 201615002418; 23.03.2016 US 201615077946
(71) Applicant: Dharma Life Sciences LLC, Sleepy Hollow, NY 10591 (US)
(72) Inventor: SASIDHAR, Jonnalagadda, Sleepy Hollow, NY New York 10591 (US); NAGPAL, Manisha Naresh, Bangalore-560050 (IN); VASUDEV, Gowthami, Bangalore 560003 (IN); AMBEKAR, Gopi Ambikapathi, Bangalore 560028 (IN); DURAIMANI, Shanthi Lakshmi, Bangalore-560005 (IN); MISHRA, Pranav Kumar, West Bengal- 713321 (IN); SUNDARAN, Sreenidhi, Bangalore- 560022 (IN); KRISHNA, Nagaranjini Honahalli, Hassan 573201 (IN)
(74) Representative: Cabinet Chaillot

(57) **Abstract**

A system for enabling a user to overcome weak behavioral traits is provided. The system comprises a behavioral traits database and a collection of games database. The behavioral traits database includes data identifying a first and second defective wirings of the brain associated with each trait. The collection of games database includes digital games, and data identifying one or more defective wirings that is affected by the game. The system is configured to receive a selection of a behavioral trait, identify the first and the second defective wirings associated with the selected trait, present at least a first digital game, which affects the identified first defective wiring, present at least a second digital game, which affects the identified second defective wiring, and enable the user to engage in the second digital game after the user has at least participated in the first digital game.

## Description

### BACKGROUND

Unless otherwise indicated herein, the materials described in this section are not prior art to the claims in this application and are not admitted to be prior art by inclusion in this section.

The subject matter in general relates to behavioral traits in humans, and more particularly but not exclusively, the subject matter is directed to a technical solution to overcome weak behavioral traits.

An individual's behavior towards others, attitudes and characteristics, defines his personality. Each individual's personality is a combination of both positive and negative traits. A person may be driven by his positive traits at times and by negative traits at other times. As a result, to classify a person into a single personality type is difficult, as there are several different behavioral traits a person can have. Behavioral traits may be influenced by genes and/or by the environment where he grew up or may have spent most of his time during the stage of brain development.

Understanding what makes people who they are has been a challenge in the world of psychology. One of the approaches used for determining the personality of a person is by way of taking a psychology based personality test. In such a test, the person answers a set of questions, and based on the answers gets a report detailing the type of personality that person may have.

Negative or weak traits in a person can lead to difficulty in achieving their life goals. Conventionally, people try to overcome their weaknesses when one understands one's weaknesses or weak traits. An individual may approach a counselor or a psychologist with a view that, the counselor or the psychologist may be able to help him address his negative or weak trait. The person may have to undergo several sessions with the counselor. Such sessions may deal with the negative trait via a broad based general (not focused at the root of the weak trait) approach, which may be effective in the short run. For example, if a person is trying to deal with stress, the person may be advised to engage in regular exercise, outdoor games, engage socially and so on, which may prove effective to an extent for the time being.

Currently, there are multiple games and applications being developed that may work in the same way as a counseling process. Even these games deal with the negative trait via a general approach. As an example, if a person is not capable of reading at good speed, the person is subjected to games related to reading to improve his speed of reading. Such approach may be effective in the short run, but may not address negative traits in a wholesome manner.

In light of the foregoing discussion there is a need for an improved technique to overcome a weak trait of personality.

### SUMMARY

In one aspect, a system for enabling a user to overcome weak behavioral traits. The system comprises a behavioral traits database and a collection of games database. The behavioral traits database includes a plurality of behavioral traits. The database further includes, for each of the behavioral traits, data identifying a first defective wiring of the brain, whose symptom is the weak behavioral trait, and data identifying a second defective wiring of the brain, whose symptom is the weak behavioral trait. The collection of games database includes a plurality of digital games, wherein for each of the digital games, the collection of games database comprises data identifying one or more defective wirings of the brain that is affected by the game. The system is configured to receive a selection of at least one of the plurality of behavioral traits, identify the first defective wiring and the second defective wiring associated with the selected behavioral trait, using the behavioral traits database, present at least a first digital game, from the collection of games database, which affects the identified first defective wiring, present at least a second digital game, from the collection of games database, which affects the identified second defective wiring, and enable the user to engage in the second digital game after the user has at least participated in the first digital game.

### BRIEF DESCRIPTION OF THE DRAWINGS

Embodiments are illustrated by way of example and not limitation in the Figures of the accompanying drawings, in which like references indicate similar elements and in which:
FIG. 1 is an exemplary architecture of an exemplary system 100 to overcome weak behavioral traits;
FIG. 2 is a block diagram of an exemplary activity module 200 to overcome weak behavioral traits;
FIG. 2A is a block diagram of an exemplary behavioral traits database 202 of the activity module 200;
FIG. 2B is a block diagram of an exemplary collection of games 204 present in the activity module 200;
FIG. 2C is a block diagram of an exemplary actions database 206 of the activity module 200;
FIG. 3 is a flowchart an exemplary method for overcoming weak behavioral traits;
FIG. 4 exemplifies choice of digital games 204 presented by the activity module 200 to a user to for rewiring 218a a defective brain wiring 216a to overcome weak trait 214a; and
FIG. 5 exemplifies the activity module 200 enabling and recording performance of exposure activities 220 in real world.

### DETAILED DESCRIPTION

### I. OVERVIEW

### II. UNDERLYING SCIENTIFIC PRINCIPLE

### III. SYSTEM ARCHITECTURE

### IV. MODULES OF SYSTEM TO OVERCOME WEAK BEHAVIORAL TRAITS

### V. METHOD FOR OVERCOMING A WEAK BEHAVIORAL TRAIT

The following detailed description includes references to the accompanying drawings, which form part of the detailed description. The drawings show illustrations in accordance with example embodiments. These example embodiments are described in enough detail to enable those skilled in the art to practice the present subject matter. However, it will be apparent to one of ordinary skill in the art that the present invention may be practiced without these specific details. In other instances, well-known methods, procedures and components have not been described in detail so as not to unnecessarily obscure aspects of the embodiments. The embodiments can be combined, other embodiments can be utilized or structural and logical changes can be made without departing from the scope of the invention. The following detailed description is, therefore, not to be taken as a limiting sense.

In this document, the terms "a" or "an" are used, as is common in patent documents, to include one or more than one. In this document, the term "or" is used to refer to a nonexclusive "or," such that "A or B" includes "A but not B," "B but not A," and "A and B," unless otherwise indicated.

### I. OVERVIEW

Embodiments provide a technical solution to overcome weak behavioral traits. The solution is based on a principle that genetic and environmental factors (may be referred to as "cause") cause defects in human brain wiring (may be referred to as "defect"), and such defects in human brain wiring express themselves in the form of weak behavioral traits (may be referred to as "symptoms"); hence, to overcome a weak behavioral trait, the defective brain wiring(s) have to be rewired, thereby providing a wholesome and long term solution to weak behavioral traits.

In an embodiment, a system is provided to overcome weak behavioral traits. The system may include an activity module. The activity module may be configured to enable the user to engage in at least one activity in a virtual environment. The activity in the virtual environment facilitates rewiring of a defective brain wiring, wherein the symptom of the defective brain wiring is the weak behavioral trait. The activity module may be further configured to provide automated instructions to gradually expose the user to situations in a real environment. The situations may be generally uncomfortable to the user as a result of the defective brain wiring. Exposure to such situation facilitates rewiring of the defective brain wiring.

The activity in the virtual environment may be a brain game. There may a plurality of levels in the brain game. Each level in the game may have to be successfully completed by the user to unlock a subsequent higher level in the game.

The exposure activity may take place in the real world environment, subsequent to the brain games. There may be a plurality of levels in the exposure activity.

### II. UNDERLYING SCIENTIFIC PRINCIPLE

Examples of behavioral traits include, but not limited to, anger, submissiveness, anxiety, sensitivity, perfectionism, pessimism and introversion. Behavioral traits are in fact symptoms or consequences of the way an individual's brain is wired. The way an individual's brain is wired is based on the joint influence of genetic and environmental factors. With regards to each kind of brain wiring, genetic and environmental factors may work together in different proportions.

As a simple example, an individual as a kid may be exposed to environmental factors such as, for an extend period of time, fulfillment of the individual's demand for having fun even though the demands were unreasonable. Said environmental factors (e.g., social interactions and cultures) along with genetic factors that may be present, cause a defective brain wiring responsible for being out of control. The individual after he grows up may exhibit a behavioral trait (symptom) of excessive shopping.

Embodiments provide a solution to rewire the defective brain wiring (addressing the defect) rather than just addressing the behavioral trait (symptom) without addressing the core defect.

It is important to identify the brain wiring that results in development of a trait. A wholesome approach to overcome or eradicate a negative or a weak behavioral trait in a person would be to implement ways to rectify defect in the brain wiring.

Brain wirings may be referred to as connections of neural pathways in the brain. These connections of the brain form beliefs, thought patterns and response patterns to external stimulation. Rewiring may be referred to as a process of creating new brain wiring and stimulating a person's existing brain wirings so that his beliefs, thought patterns and responses to external stimulation change for the better.

### III. SYSTEM ARCHITECTURE

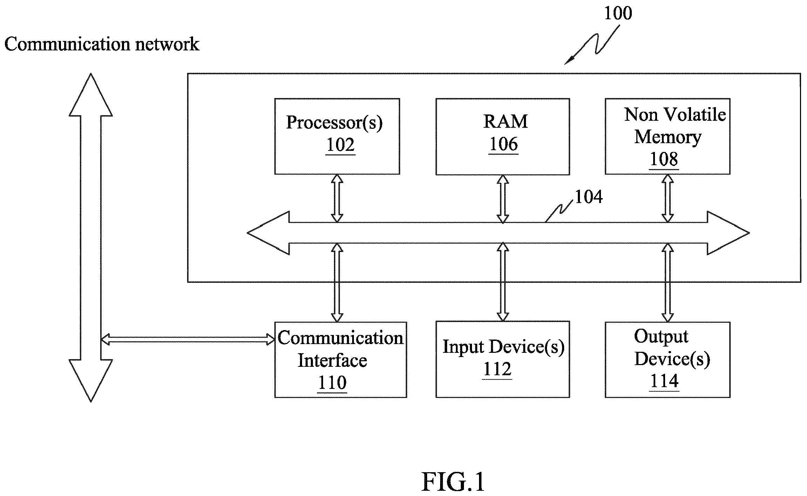

Referring to the figures, more particularly to FIG. 1, an exemplary architecture of an exemplary system 100 to overcome weak behavioral traits is provided. The system 100 include one or more processors 102, a bus system 104, a random access memory 106, a disk drive or non-volatile memory 108, a communication interface 110, input device(s) 112 and output device(s) 114. Further, functionality of the system 100 may be distributed across multiple devices that may be located remotely from each other.

The processor 102 may be any hardware which returns output by accepting signals, such as electrical signals as input. In one embodiment, processors 102 may include one or more computer processing units (CPUs). The processor(s) 102 may communicate with a number of peripheral devices via the bus system 104. The processor(s) 102 may be implemented as appropriate in hardware, computer-executable instructions, firmware, or combinations thereof. Computer-executable instruction or firmware implementations of the processor(s) 102 may include computer-executable or machine-executable instructions written in any suitable programming language to perform the various functions described.

Communications interface 110 may provide an interface to other communication networks and devices. The input devices 112 may include all possible types of devices and mechanisms for inputting information to system 100. The output devices 114 may include all possible types of devices and mechanisms for outputting information from the system 100. The system 100 may include memory that may store data and program instructions that are loadable and executable on the processor(s) 102, as well as data generated during the execution of these programs. The memory may be volatile, such as random access memory 106 and/or a disk drive or non-volatile memory 108.

### IV. MODULES OF SYSTEM TO OVERCOME WEAK BEHAVIORAL TRAITS

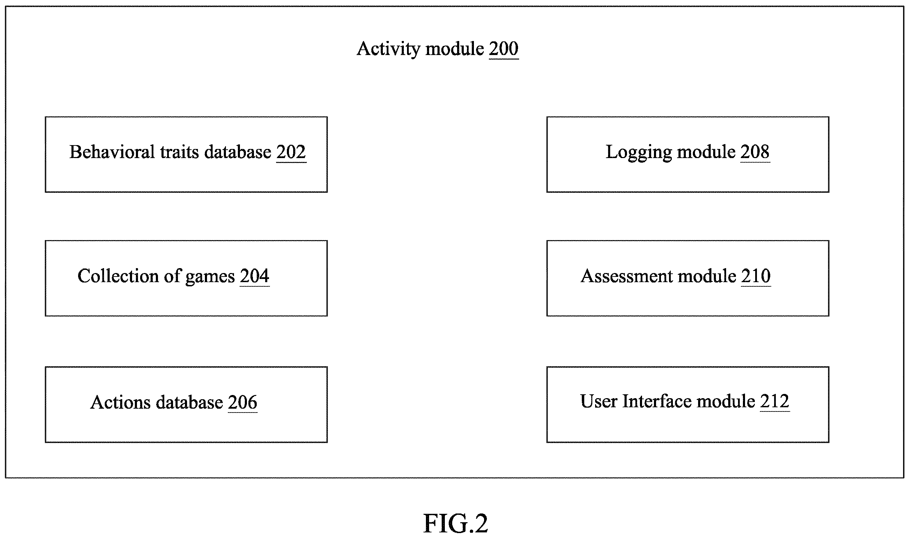

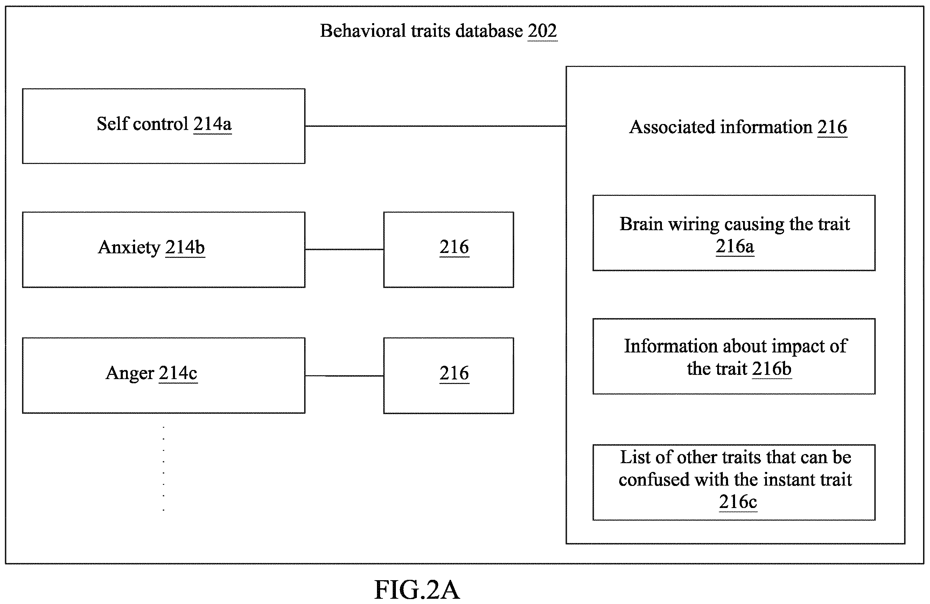

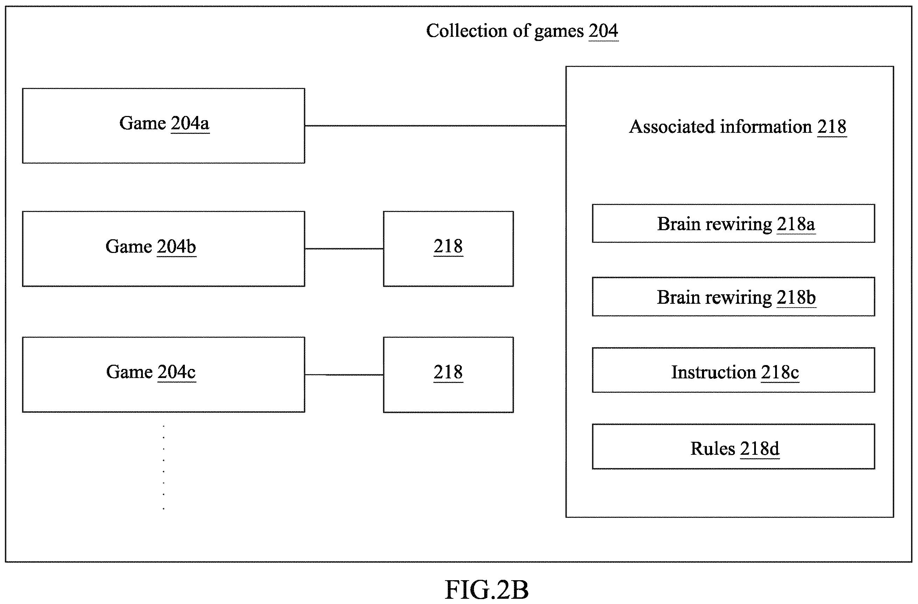

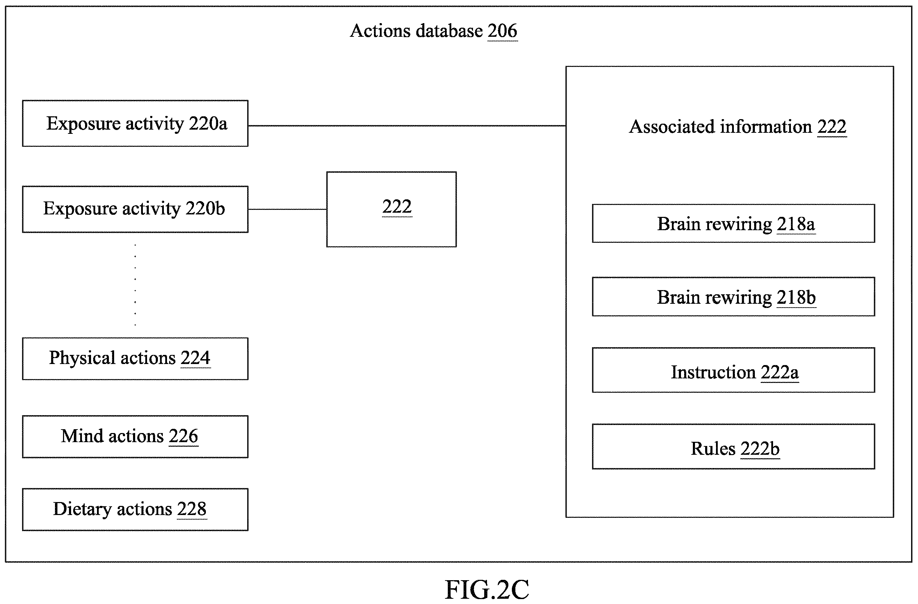

Referring to the figures, and more particularly to FIG. 2, the system 100 includes various modules for enabling users to overcome their weak behavioral traits. The system 100 includes an activity module 200. The activity module 200 may be a gaming module or an application module. The activity module 200 may be hosted by a communication device that enables a user to play games. Examples of such communication devices may include smart phone, tablet, notebook, laptop and desktop computer, among other devices.

The activity module 200 may include a behavioral traits database 202, a collection of games 204, an actions database 206, a logging module 208, an assessment module 210 and a user interface module 212.

### Behavioral traits database 202

Referring to FIG. 2A, in an embodiment, the behavioral traits database 202 may include a list of behavioral traits 214. Examples of behavioral traits 214 include, but not limited to, self control 214a, anxiety 214b and anger 214a, among others. The traits database 202 may further include, with respect to one or more of the behavioral traits, information associated 216 with the trait 214. The associated information 216 may include information corresponding to the brain wiring (defect) 216a causing the trait (symptom of the defect), information about impact 216b of the trait 214 on a person, and a list 216c comprising one or more traits 214 that may be confused with the instant trait, among other information. Some or all of the associated information 216 may be displayed to the user of the activity module 200.

In an embodiment, the information corresponding to the brain wiring (defect) 216a causing the trait 214 enables the activity module 200 to select games, activities or actions to overcome respective trait 214.

Examples of defective brain wiring 216a include, but not limited to, lack of self control.

In an embodiment, displaying the information corresponding to the brain wiring (defect) 216a causing the trait 214 helps the user in understanding the defect(s) responsible for his weak behavioral trait, thereby motivating the user to follow the instructions provided by the activity module 200.

In an embodiment, the activity module 200 displays the information corresponding to the brain rewiring that the activity module 200 is attempting to achieve to overcome the weak behavioral trait 214, thereby enabling the user to understand the actual goal he will be instructed to work towards, which in turn can overcome his weak behavioral trait 214.

In an embodiment, the activity module 200 displays the information corresponding to the causes (genetic or environmental) that resulted in the defective brain wiring (defect) 216a, which in turn resulted in the trait 214, thereby enabling the user to relate to the cause, defect and symptom of the defect, which results in a wholesome experience while working on the weak behavioral trait chosen by the user.

### Collection of games 204

The activity module 200 includes a collection of digital games 204, which are played in a virtual world. Each of the games 204 is used by the activity module 200 based on the brain rewiring, which the game 204 is capable of achieving.

Referring to FIG. 2B, in an embodiment, each game 204a, 204b, 204c... (may be referred to as game 204 or games 204) may include associated information 218. As an example, a game 204a may be capable of achieving more than one type of brain rewiring 218a, 218b. Each game 204 includes data indicating the brain rewiring(s) 218a, 218b it is capable of achieving, so that the activity module 200 can opt to present a choice of one or more games, which have the capability to achieve the brain rewiring, which the activity module 200 is attempting to achieve.

The associated information 218 may further include instructions 218c to be followed by the user to play the game 204. The associated information 218 may additionally include one or more rules 218d. Examples of rules include rules for playing each game, rules to proceed to a higher level, scoring method in each level and time assigned to each level to reach the end of the level, among others.

In an embodiment, a higher level of the digital game 204 is unlocked based on predefined criteria.

In an embodiment, the higher level is unlocked upon repeatedly playing a previous level in the digital game 204 for a predefined number of times or duration of time.

### Actions database 206

The actions database 206 includes information corresponding to a plurality of exposure activities or sets of exposure activities. Exposure activities may include activities that are performed in a real world environment, as opposed to the digital games played in the virtual world (ex: games included in the collection of games 204). Examples of exposure activities include, but not limited to, counting the number of restaurants that are on the way back from one's office to home, interacting with a neighbor, interacting with a friend's friend, asking them their names, addresses, hobbies and profession, among others.

Referring to FIG. 2C, each of the exposure activities 220a, 220b ... (may be referred by numeral 220) or sets of exposure activities 220a, 220b ... (may be referred by numeral 220) is used by the activity module 200 based on the brain rewiring, which the exposure activity 220 is capable of achieving.

Each exposure activity 220 may include associated information 222. As an example, an exposure activity 220a may be capable of achieving more than one type of brain rewiring 218a, 218b. Each exposure activity 220 includes data indicating the brain rewiring(s) 218a, 218b it is capable of achieving, so that the activity module 200 can opt to present a choice of one or more exposure activities 220, which has the capability to achieve the brain rewiring, which the activity module 200 is attempting to achieve.

The associated information 222 may further include instructions 222a to be followed by the user to perform the exposure activity 220 or a set of exposure activity 220. The associated information 222 may additionally include one or more rules 222b. Examples of rules include rules to proceed to a higher level, scoring method in each level and time assigned to each level to reach the end of the level, among others.

In an embodiment, the action database 220 further includes plurality of physical actions 224, mind actions 226 and dietary actions 228, one or more of which may be presented to a user who is trying to overcome a weak behavioral trait 214.

Example of physical actions 224 includes one or more physical activities, such as exercises and physical postures, among others. Example of mind actions 226 may include meditation. Example of dietary actions 228 may include adding specific food to one's diet or deleting specific food from the diet. As a further example, drinking black tea may be a dietary action and cutting down on rice consumption may be another dietary action.

### Logging module 208

In an embodiment, the logging module 208 enables logging of the activities (ex: games, exposure activities and other actions) performed by the user. The logging module 208 may record information corresponding to the activities performed by the user. The information may include one or more of, time of performance, date of performance, quantum of performance, success or failure consequent to an attempt to perform, and extent of success in performing the action, among others. The user may provide input that an activity has been performed. Such input may be used to log the activity in the logging module 208. Alternatively, the logging module 208 may be configured to automatically log the performance of an activity upon detection that the activity has been performed.

### Assessment module 210

The assessment module 210 of the activity module 200 is configured to determine the performance of a user in each activity the user performs. The assessment module 210 may determine the user's score for each game and at each level in a game based on the log available in the logging module 208. Further, the assessment module 210 analyzes performance of the user in the exposure activities and determines scores of each level of exposure activity based on the log that is present in the logging module 208. The assessment module 210 may also determine status of the one or more behavioral trait 214 or the brain wiring causing the trait 202, after engaging in the activities, based on performance of activities suggested by the activity module 200.

### User interface module 212

The user interface module 212 may be configured to receive input from the user and display content to the user. The content displayed, can be, as an example, games, images, instructions, rules, information, haptic feedback and sound, among others.

### V. METHOD FOR OVERCOMING A WEAK BEHAVIORAL TRAIT

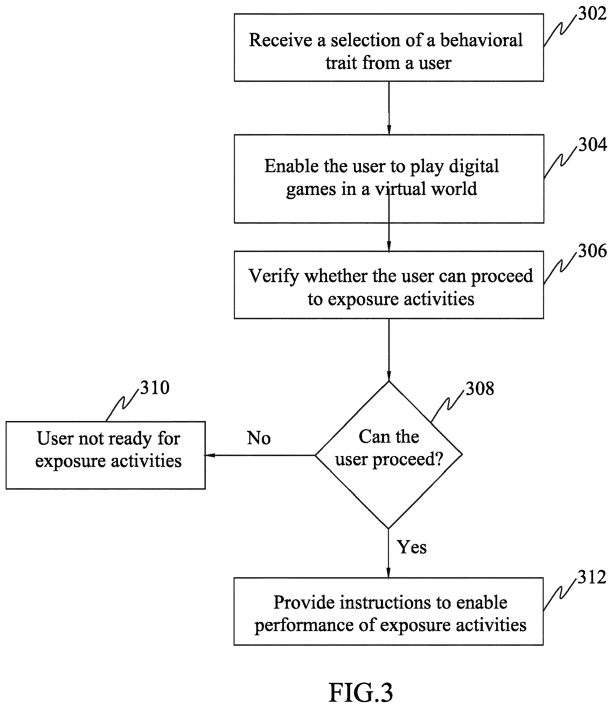

Now referring more particularly to FIG. 3, a method is provided for overcoming a weak behavioral trait 214. A selection of a behavioral trait 214, which a user wishes to work on, is received at step 302. At step 304, a user is enabled to participate/play in at least one digital game 204a, which is played in a virtual world. The digital game 204a is instrumental in rewiring 218a a defective brain wiring 216a, whose symptom is exhibition of said weak behavioral trait 214. At step 306, verification is made to determine whether the user can proceed to perform exposure activities 220. In case it is determined at step 308 that user cannot proceed, then it is established that the user may not be ready for exposure activities (step 310). On the other hand if it is determined at step 308 that user can proceed, then at step 312 instructions are provided to the user to enable the user to perform exposure activity/activities 220a. Preferably, the user is allowed to perform or instructed to begin performing exposure activities 220 upon participating in the digital game 204a at least to a predefined extent, which is determined based on the desired rewiring. The exposure activity 220a is instrumental in rewiring 218a a defective brain wiring 216a, whose symptom is exhibition of said weak behavioral trait 214.

Referring to the step (302) of receiving a selection of a behavioral trait 214, the user may be provided an option to select a behavioral trait that he would be like to overcome. The activity module 200 may provide or recommend an option to select at least one weak behavioral trait to work on. The user may select at least one behavioral trait 214 that the user thinks he needs to work on, from a list of behavioral traits that may be displayed to the user via the user interface module 212. Alternatively, the user may input answers in response to a set of questions displayed to the user, which may determine what type of weak behavioral trait 214 the user may have. Upon selection of a behavioral trait 214, the activity module 200 may also display a list 216c of other behavioral traits that can be confused with the instant behavioral trait 214, so that the user can select a more appropriate behavioral trait 214, which he desires to work on. The activity module 200 may further display the information corresponding to the brain wiring (defect) 216a causing the trait behavioral 214. The activity module 200 may additionally display information about impact 216b of the behavioral trait 214 on the user. In addition, the activity module 200 may display information corresponding to the brain rewiring 218a that the activity module 200 is attempting to achieve to overcome the weak behavioral trait 214. Furthermore, the activity module 200 may display the information corresponding to the causes (genetic or environmental) that resulted in the defective brain wiring (defect) 216a.

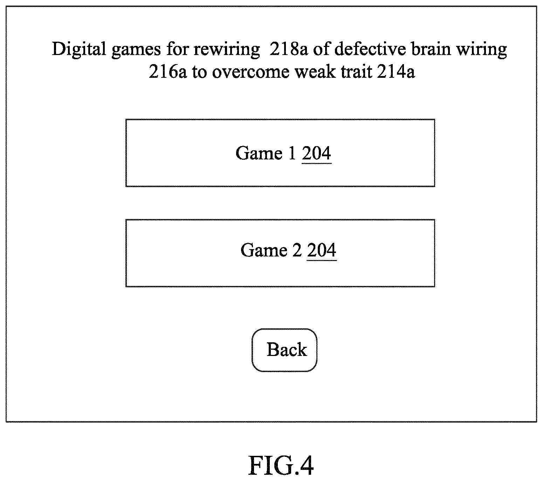

Referring to the step (304) of enabling the user to play digital game(s) 204a in a virtual world, the activity module 200 may select one or more digital game(s) 204 based on the capability of the digital game(s) 204 to achieve the desired brain rewiring 218a. Referring to FIG. 4, the activity module 200 may provide a choice (one or more games) of games 204, from which the user may select. Each of the games has the capability to achieve the desired brain rewiring 218a of the defective brain wiring 216a to overcome the weak behavioral trait 214. The activity module 200 may even indicate the effectiveness of each of the games in achieving the desired brain rewiring 218a. The activity module 200 may select the games 204, to present them as a choice to the user, based on the associated information 218, which includes information indicating the brain rewiring (ex: 218a, 218b), which the game 204 is capable of achieving.

In an embodiment, the activity module 200 is configured to recommend a plurality of games 204, wherein the recommendation indicates each of the games' 204 effectiveness to achieve the desired brain rewiring 218a.

The activity module 200 allows the user to play the selected game 204. The logging module 208 may log the performance of the user. The assessment module 210 may determine the performance scores using the log in the logging module 208. Further, based on the rules 218d, the user is either allowed or denied access to other levels of the game 204.

Referring to step 306, the activity module 200 verifies whether the user can proceed to perform exposure activities 220. Preferably, the activity module 200 recommends exposure activities 220 after the user has performed to a desired extent in the digital game(s) 204. Desired extent of performance can be, as an example, levels completed, score reached and duration over which the game 204 was played.

Referring to step 312, if it is determined that user can proceed, then instructions are provided to the user to enable the user to perform exposure activity/activities 220a. Preferably, the user is allowed to perform or instructed to begin performing exposure activities 220 upon participating in the digital game 204a at least to an extent, which is determined based on the desired rewiring. The exposure activity 220a is instrumental in rewiring 218a a defective brain wiring 216a, whose symptom is exhibition of said weak behavioral trait 214.

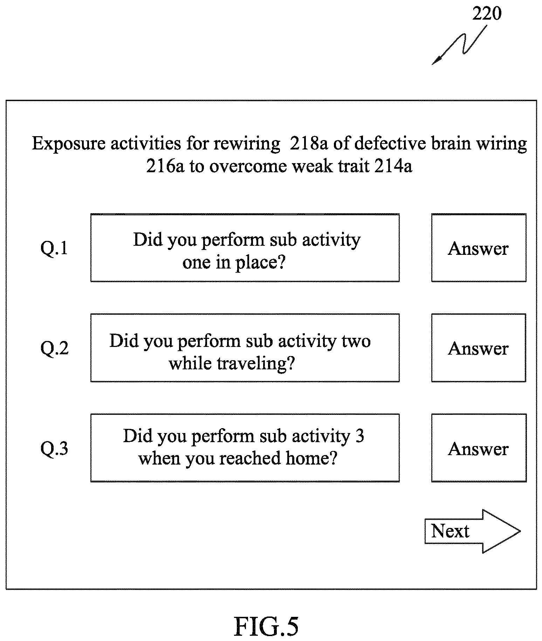

The activity module 200 may select one or more exposure activities 220 based on the capability of the exposure activities 220 to achieve the desired brain rewiring 218a. Referring to FIG. 5, the activity module 200 may provide a choice (one or more exposure activity or sets of exposure activities) of exposure activities 220 (choices not shown), from which the user may select. Each of the sets has the capability to achieve the desired brain rewiring 218a of the defective brain wiring 216a to overcome the weak behavioral trait 214. The activity module 200 may even indicate the effectiveness of each of the sets of exposure activities 220 in achieving the desired brain rewiring 218a. The activity module 200 may select the exposure activities or sets of exposure activities 220, to present them as a choice to the user, based on the associated information 222, which includes information indicating the brain rewiring (ex: 218a, 218b), which the exposure activities or sets of exposure activities 220a is capable of achieving.

In an embodiment, the activity module 200 is configured to recommend a plurality of exposure activities or sets of exposure activities 220, wherein the recommendation indicates each of the exposure activities' or sets of exposure activities' 220 effectiveness to achieve the desired brain rewiring 218a.

The activity module 200 instructs the user to perform the exposure activities 220. The logging module 208 may log the performance of the user. The assessment module 210 may determine the performance scores using the log in the logging module 208. Further, based on the rules 218d, the user is either allowed or denied access to other levels of the exposure activities or sets of exposure activities 220.

In an embodiment, at least two defective wirings 216 result in the user exhibiting a weak behavioral trait 214. Hence, the activity module 200 suggests and enables participation in digital games 204 directed at rewiring 218 the two defective wirings 216. A first set of digital game(s) 204 may be directed at rewiring 218 a first defective brain wiring 216. A second set of digital game(s) 204 may be directed at rewiring 218 a second defective brain wiring. The activity module 200 suggests participation in the second set of digital game(s) 204 only after the user has participated in the first set of digital game(s) 204 at least to a desired extent.

It may be noted that, attempting to rewire the second defective wiring 216 without at least having rewired the first defective wiring 216 to an extent may not result in effective rewiring of the defective wirings 216, and thereby may not result in alleviating the weak behavioral trait 214.

In an embodiment, the activity module 200 provides automated instructions to the user to enable the user to perform at least one exposure activity 220. The exposure activity 220 is directed at rewiring the first defective wiring 216 of the brain. The instructions to engage in the exposure activity 220 directed at rewiring the first defective wiring 216 is provided after the user has engaged in the first set of digital game(s) 204 directed at rewiring 218 the first defective brain wiring 216. Likewise, the activity module 200 provides automated instructions to the user to enable the user to perform at least one exposure activity 220 directed at rewiring the second defective wiring 216 of the brain. The instructions to engage in the exposure activity 220 directed at rewiring the second defective wiring 216 is provided after the user has engaged in the second set of digital game(s) 204 directed at rewiring 218 the second defective brain wiring 216.

In an embodiment, the system may provide the user with options to choose from one or more traits, such as, introversion, anxiety, depression, low empathy, social anxiety, perfectionism, lack of self control, sensitivity, pessimism, anger, submissiveness and high empathy.

### INTROVERSION

In case of introversion, a first defective wiring results in tendency to be selective in information gathering. The virtual games and the exposure activities are directed to rewire the brain to increase the ability to gather information concurrently from multiple sources. A second defective wiring of the brain results in reduced speed of verbal response to open ended stimuli. The virtual games and the exposure activities are directed to increase speed of verbal response to open ended stimuli.

### ANXIETY

In case of anxiety, a first defective wiring of the brain results in an individual giving priority to threatening information in the environment. The virtual games and the exposure activities are directed to increase ability to focus on or prioritize non threatening information in environment. A second defective wiring of the brain results in an individual failing to make predictions about predictable events. The virtual games and the exposure activities are directed to increase the ability to make predictions based on available facts. A third defective wiring of the brain results in an individual believing they do not have control of situation. The virtual games and the exposure activities are directed to increase ability to gain control of the situation by choosing the best solution (as may be perceived) available.

### DEPRESSION

In case of depression, a first defective wiring of the results in an individual failing to disengage from negative self referential thoughts. The virtual games and the exposure activities are directed to increase ability to disengage from negative self referential thoughts and engage in positive self referential thoughts. A second defective wiring of the brain results in negative memories being stronger than positive memories and the number of negative memories outweighing the number of positive memories. The virtual games and the exposure activities are directed to strengthen and increase the number of positive memories.

### LOW EMPATHY

In case of low empathy, a first defective wiring of the brain results in difficulty in reading others' social cues. The virtual games and the exposure activities are directed to increase the ability to read others' social cues. A second defective wiring results in giving exceedingly high priority to self than others. The virtual games and the exposure activities are directed to increase ability to weigh between self and others and determine who needs to get more priority depending on the situation.

### SOCIAL ANXIETY

In case of social anxiety, a first defective wiring of the brain results in prioritizing threatening information in social environments. The virtual games and the exposure activities are directed to increase the ability to focus or prioritize on non threatening information in social environments. A second defective wiring results in fear of negative evaluation by others. The virtual games and the exposure activities are directed to increase ability to balance between positive and negative evaluation by other people and decide if the experience is good or not.

### PERFECTIONISM

In case of perfectionism, a first defective wiring results in prioritizing on errors in a task more than the completion of the task. The virtual games and the exposure activities are directed to increase the ability to focus on the completion of the task. A second defective wiring results in inability to weigh between the errors in the task and the completion of the task. The virtual games and the exposure activities are directed to increase the ability to weigh between the errors in the task and the completion of the task.

### SELF CONTROL

In case of self-control, a first defective wiring of the brain results in a tendency to be biased towards short term gratification. The virtual games and the exposure activities are directed to increase the ability to notice the benefits of long term outcome. A second defective wiring results in failure to weigh between short term gratification and long term outcome. The virtual games and the exposure activities are directed to increase ability to weigh between short term gratification and long term outcome.

### SENSITIVITY

In case of sensitivity, a first defective wiring of the brain results in amplifying one stimulus over other stimuli. The virtual games and the exposure activities are directed to increase the ability to disable the amplification of one stimulus over other stimuli. A second defective wiring of the brain, wherein the second defective wiring results in inability to consider and weigh all the stimuli which is required for them to give an appropriate response. The virtual games and the exposure activities are directed to increase the ability to consider and weigh all the stimuli which are required for them to give appropriate response.

### PESSIMISM

In case of pessimism, a first defective wiring of the brain results in giving priority to thoughts/facts that predict negative outcomes. The virtual games and the exposure activities are directed to increase the ability to give priority to or focus on thoughts/facts that predict a positive outcome. A second defective wiring of the brain results in inability to weigh between positive and negative outcomes based on facts. The virtual games and the exposure activities are directed to increase ability to weigh between positive and negative outcomes based on facts.

### ANGER

In case of anger, a first defective wiring of the brain results in bias towards noticing unfair attributes in a situation or an event. The virtual games and the exposure activities are directed to increase the ability to identify the fair attributes of a situation. A second defective wiring results in inability to weigh between fair and unfair attributes of a situation. The virtual games and the exposure activities are directed to increase ability to weigh between fair and unfair attributes of a situation.

### SUBMISSIVENESS

In case of submissiveness, a first defective wiring of the brain results in inclination towards the fear of retaliation and/or losing good impression. The virtual games and the exposure activities are directed to increase the ability to focus more on the benefits of disagreeing. A second defective wiring results in inability to weigh between the benefits of disagreeing and the fear of retaliation and/or losing good impression. The virtual games and the exposure activities are directed to increase ability to weigh between the benefits of disagreeing and the fear of retaliation and/or losing good impression.

### HIGH EMPATHY

In case of high empathy, a first defective wiring of the brain results in an individual being biased to mirror the emotional state of others. The virtual games and the exposure activities are directed to increase the ability to disengage the individuals themselves from others' emotions. A second defective wiring of the brain results in inability to weigh between the importance of self versus others. The virtual games and the exposure activities are directed to increase ability to weigh between the importance of self versus others, based on facts.

Additionally, the activity module 200 may provide instruction to perform mind actions, physical actions and dietary actions.

Each of the physical and dietary actions may have effect on one or more hormones corresponding to one or more traits, among others. While executing the physical action the energy expended in the body is more compared to the energy expended while playing brain game. Similarly, the mind action may involve thinking about the specific trait in a way which may change the structure of the brain by changing the Axons, Receptors and/or the number of Neurons. The dietary action may include consuming specific foods which changes one or more hormone levels.

The exposure, mind, physical and dietary activities are carried out in a real environment. Automated instructions are provided as to when and how the activities may have to be carried out. Further, there may be instructions that one or more of the real world activities may have to be carried out simultaneously with the brain games and one or more of the activities may have to be carried out prior to or after engaging in brain game (digital games) and performing to a desired extent.

In an embodiment, upon selecting a behavioral trait, the activity module 200 provides a scale operable by the user to select a position on the scale. The position may be indicative of the user's perception of the behavioral trait. Improvement in the behavioral trait resulting from the rewiring of the brain achieved by the performance of the activities (virtual games or exposure activities) enabled by the activity module 200 is reflected on the scale. Degradation of the improvement in the behavioral trait resulting from the degradation of the rewiring of the brain due to premature halt in performance of the activities (virtual games or exposure activities) enabled by the activity module 200 is also reflected on the scale.

The games and the exposure activities change the neural connections in the brain. When these activities are carried out repeatedly, new neural connections are formed

(brain plasticity) but if one stops carrying out these activities prematurely then slowly the neural connections die.

The processes described above is described as sequence of steps, this was done solely for the sake of illustration. Accordingly, it is contemplated that some steps may be added, some steps may be omitted, the order of the steps may be re-arranged, or some steps may be performed simultaneously.

The example embodiments described herein may be implemented in an operating environment comprising software installed on a computer, in hardware, or in a combination of software and hardware.

Although embodiments have been described with reference to specific example embodiments, it will be evident that various modifications and changes may be made to these embodiments without departing from the broader spirit and scope of the system and method described herein. Accordingly, the specification and drawings are to be regarded in an illustrative rather than a restrictive sense.

Many alterations and modifications of the present invention will no doubt become apparent to a person of ordinary skill in the art after having read the foregoing description. It is to be understood that the phraseology or terminology employed herein is for the purpose of description and not of limitation. It is to be understood that the description above contains many specifications, these should not be construed as limiting the scope of the invention but as merely providing illustrations of some of the personally preferred embodiments of this invention.

## Claims

1. A system for enabling a user to overcome weak behavioral traits, the system comprising:
a behavioral traits database comprising a plurality of behavioral traits, the database further comprising for each of the behavioral traits:
data identifying a first defective wiring of the brain, whose symptom is the weak behavioral trait; and
data identifying a second defective wiring of the brain, whose symptom is the
weak behavioral trait; and
a collection of games database comprising a plurality of digital games, wherein for each of the digital games, the collection of games database comprises data identifying one or more defective wirings of the brain that is affected by the game;
wherein the system is configured to:
receive a selection of at least one of the plurality of behavioral traits;
identify the first defective wiring and the second defective wiring associated with the selected behavioral trait, using the behavioral traits database;
present at least a first digital game, from the collection of games database, which affects the identified first defective wiring;
present at least a second digital game, from the collection of games database, which affects the identified second defective wiring; and
enable the user to engage in the second digital game after the user has at least participated in the first digital game.

2. The system according to claim 1, wherein the collection of games database further comprises, for each of the digital games, data indicating effectiveness in achieving desired rewiring of the one or more defective wiring affected by the digital game, wherein the system is configured to:
present a plurality of digital games, from the collection of games database, which affect the identified first defective wiring;
present a plurality of digital games, from the collection of games database, which affect the identified second defective wiring;
indicate, to the user, the effectiveness of the presented digital games in achieving the desired rewiring of the first defective wiring; and
indicate, to the user, the effectiveness of the presented digital games in achieving the desired rewiring of the second defective wiring.

3. The system according to claim 1, wherein the collection of games database further comprises, for each of the digital games, data indicating effectiveness in achieving desired rewiring of the one or more defective wiring affected by the digital game, wherein the system is configured to:
present a plurality of digital games, from the collection of games database, which affect the identified first defective wiring;
present a plurality of digital games, from the collection of games database, which affect the identified second defective wiring;
provide indication recommending, to the user, at least one of the presented plurality of digital games, at least based on the effectiveness of the presented digital games in achieving the desired rewiring of the first defective wiring; and
provide indication recommending, to the user, at least one of the presented plurality of digital games, at least based on the effectiveness of the presented digital games in achieving the desired rewiring of the second defective wiring.

4. The system according to claim 1 further comprising an actions database comprising instructions corresponding to a plurality of exposure activities, which are to be performed in real-world environment, wherein for each of the exposure activities, the actions database comprises data identifying one or more defective wirings of the brain that is affected by the exposure activities, wherein the system is configured to:
present instructions corresponding to at least one of the exposure activities,
from the actions database, which affect the identified first defective wiring; and
present instructions corresponding to at least one another exposure activities,
from the actions database, which affect the identified second defective wiring.

5. The system according to claim 4, wherein the system is configured to:
present the instructions corresponding to the at least one of the exposure activities,
which affect the identified first defective wiring, after the user has engaged in the first digital game at least to a predefined extent; and
present the instructions corresponding to the at least one other exposure activities,
which affect the identified second defective wiring, after the user has engaged in the second digital game at least to a predefined extent.

6. The system according to claim 4, wherein the activity module is further configured to:
provide a scale operable by the user to select a position on the scale, wherein the position is indicative of the user's perception of the behavioral trait;
reflect on the scale improvement in the behavioral trait resulting from rewiring of the first and second defective wirings; and
reflect on the scale degradation of the improvement in the behavioral trait resulting from degradation of the rewiring due to premature halt in engaging in the presented virtual games and the exposure activities.

7. The system according to claim 1, wherein, if the weak behavioral trait is anxiety, the first digital game is directed to rewire the first defective wiring, which results in an individual giving priority to threatening information in the environment, to increase ability of the user to focus on or prioritize non-threatening information in the environment; and
the second digital game is directed to rewire the second defective wiring, which results in an individual failing to make predictions about predictable events, to increase ability of the user to make predictions based on available facts.

8. The system according to claim 1, wherein, if the weak behavioral trait is sensitivity, the first digital game is directed to rewire the first defective wiring, which results in amplifying one stimulus over other stimuli, to increase ability of the user to disable amplification of one stimulus over other stimuli; and
the second digital game is directed to rewire the second defective wiring, which results in inability to consider and weigh all stimuli which are required for to give an appropriate response, to increase ability of the user to consider and weigh all stimuli which are required to give appropriate response.

9. The system according to claim 1, wherein, if the weak behavioral trait is perfectionism,
the first digital game is directed to rewire the first defective wiring, which results in prioritizing on errors in a task more than completion of the task, to increase ability of the user to focus on the completion of the task; and
the second digital game is directed to rewire the second defective wiring, which results in inability to weigh between errors in a task and completion of the task, to increase ability of the user to weigh between the errors in the task and the completion of the task.

10. The system according to claim 1, wherein the behavioral traits database further comprises, for at least one of the behavioral traits, data identifying one or more additional behavioral traits, which may be confused with said at least one of the behavioral traits, wherein the system is configured to, present to the user said one or more additional behavioral traits in response to a selection of said at least one of the behavioral traits.
